# EUROPEAN PATENT SPECIFICATION

(11) **EP 0 773 789 B1**
(45) Date of publication and mention of the grant of the patent: **28.03.2007**
(21) Application number: 95904529.5
(22) Date of filing: 22.12.1994
(51) Int. Cl.: A61K 38/17, C07K 1/12

(54) **Use of MHC xenoantigen in the manufacture of a medicament for the treatment of cancer**
Verwendung von MHC-Xenoantigenes zur Herstellung eines Arzneimittels für eine therapeutische Behandlung von Krebs
Utilisation de xénoantigènes CMH pour la fabrication d'un médicament pour le traitement du cancer

(30) Priority: 05.08.1994 IT MI941713
(43) Date of publication of application: 21.05.1997
(73) Proprietor: Applied Research Systems N.V., Curaçao (AN)
(72) Inventor: Turiano, Angela, 20100 Milano (IT)
(74) Representative: Gislon, Gabriele
(86) International application number: PCT/EP1994/004290
(87) International publication number: WO 1996/004005

(56) References cited:
- EP-A- 0 563 627
- EP-A- 0 569 678
- WO-A-93/14126
- WO-A-94/01130
- WO-A-94/13320
- JOURNAL OF IMMUNOLOGICAL METHODS, vol. 112,no. 1, 9 August 1988 AMSTERDAM, THE NETHERLANDS, pages 133-138, M. LABETA ET AL. 'Solubilisation effect of Nonidet P-40, Triton X-100 and CHAPS in the detection of MHC-like glycoproteins.'

## Description

### Field of the invention

The present invention concerns the use of xenoantigens, which are useful for stimulation of the immune system, particularly for cancer therapy, i.e., in the manufacture of a medicament for treatment of cancer in man and in mammals in general. More specifically, the use of major histocompatibility complex (MHC) antigens extracted from animal tissues and with the same molecular weight (between 10,000 and 50,000 daltons).

### Background of the art

In the treatment of cancer, in view of the results up to now obtained with synthetic drugs and chemical and physical therapies, investigators have sought ways to develop systems to stimulate the immune response of the organism against the proliferation of cancer cells. A factor called AF2 (alpha-fetoprotein), obtained from extracts of lambs and sheep embryos, has been used in the antitumor support therapy as an analgesic and biological antiemetic (Schweiz. Rundsch. Med. Prax. 1990, 79(16): 498-502). An antitumor polypeptide obtained from macrophage-like human cells and with a molecular weight of 47,010 daltons is also known.

In the Italian patent application no. MI 93A001369 filed in the name of the present applicant, it is described how histocompatibility antigens and ubiquitine or associated molecules administered to tumor-bearing subjects stimulate a cell-mediated and/or humoral immune response that can produce inhibition of the growth of the tumor cells and a regression of the neoplastic disease. It is known that MHC antigens (which are ubiquitously present on the cells) perform a function of vital importance for the regulation of the immune system by taking part in the mechanism that allows the immune cells to differentiate foreign antigens (non-self) from antigens of the individual (self). The antigens and more in general the histocompatibility molecules are coded by a group of genes localized in a wide region of a single chromosome.

Three main classes of genes are recognized: *class I,* which includes genes that codify for antigens and molecules responsible for rejection and transplants and destruction of altered autologous cells; *class II,* which includes genes that codify for the surface structures that interfere in the cooperation between B and T lymphocytes and macrophages; and *class III,* which includes genes that codify for some fractions of the "complement system" and the synthesis of a serum protein (protein Ss).

The MHC antigens of class I are made up of two polypeptide chains that are not covalently linked. The largest chain, responsible for allogenicity, has a molecular weight of about 43,000 daltons. The smallest chain, identified with the β-2 microglobulin, has a molecular weight of about 13,000, is not codified by the MHC system, and is always the same for each molecule of class I. The two polypeptide chains are covalently associated to an unchanging chain of about 33,000 daltons.

The MHC antigens of class II are characterized by two glycoprotein chains - one with a molecular weight of 34,000 daltons (called alpha), and the other with a molecular weight of 29,000 daltons (called beta).

The MHC antigens are usually isolated after the cells have been destroyed by sonication, treatment with liquid nitrogen, or freezing/defrosting. Once the membranes have been prepared, histocompatibility molecules are extracted by means of a variety of detergents or by proteolysis.

Salts of high ionic strength are also used for the extraction of MHC antigens from vital cells, but not from cell membranes. The greatest amount of histocompatibility antigens is obtained consistently with detergents, such as Nonidet P40 (see for an example, A. Dautigny et al, *Biochimica et Biophysica Acta,* 298:783-789, 1973). In this case, it is possible to separate the various MHC specificities by electrophoresis at a pH gradient.

WO9314126 discloses a major histocompatibility complex class II antigen for use as a vaccine against an immunodeficiency virus in particular HIV.

WO9401130 discloses a major histocompatibility complex class I antigen for use as a vaccine against an immunodeficiency virus in particular HIV.

EP 563 627 discloses a method to treat cancer using immunoreaction specific to tumor antigens expressed on the surface of tumor cells. In particular a non-classical histocompatibility class 1 antigen is administered as an immunotherapy.

EP 569 678 discloses the use of tumor cell into which at least two genes encoding MHC proteins of different haplotypes have been inserted, and wherein at least one of said MHC proteins has the same haplotype as a haplotype of the individual to be treated.

EP 569 678 discloses the use of oligopeptides derived from hypervariable region of the beta chain of an MHC Class II molecule for the treatment of deleterious immune responses, such as autoimmunity and allergies.

Labeta et al., J Immunol Methods. 1988;112(1):133-8, disclose methods of solubilisation using different detergents, the solubilisation being followed by immunoprecipitation and SDS PAGE analysis.

Dautigny et al., Biochim Biophys Acta. 1973. 298(4):783-9, disclose a method of purification of HL-A antigens from human platelets. The method describes consists of a solubilisation step using NP-40 as detergent followed by a dialysis step.

### Summary of the invention

It has been found that the immune response of an organism affected by a neoplasm improves considerably when MHC antigens extracted from animal tissues, serum or cells of various species (xenoantigens) are administered. Such an effect is even more pronounced and long-lasting if xenoantigens of different origin are alternated.

### Objects of the invention

The object of the present invention is therefore the use of MHC xenoantigens for stimulation of the immune system, said MHC molecules being susceptible to be extracted from animal or human tissues, serum or cells.

The term *histocompatibility molecules* herein refers to MHC antigens and all the molecules that are codified analogously to histocompatible antigens by the corresponding genetic MHC loci, as well as their associated molecules obtained by extraction, and also refers to antigens of red blood cells.

The term *histocompatibility molecules of different origin* herein refers to MHC molecules obtained from different organisms or species, or originating from different batches of tissues, serums or cells.

As stated above, histocompatibility molecules useful for the invention are usually obtainable by extraction from animal tissues, together with their associated molecules. The histocompatibility antigens and molecules constitute the true active ingredient, whereas their associated molecules are extracted together with the antigens and presumably act as carriers for the histocompatibility molecules.

The histocompatibility antigens and molecules used for the present invention were prepared from mammalian liver tissue, usually homogenized veal or goat liver.

### Brief description of the drawings

The invention will now be further described with reference to enclosed drawings, in which:

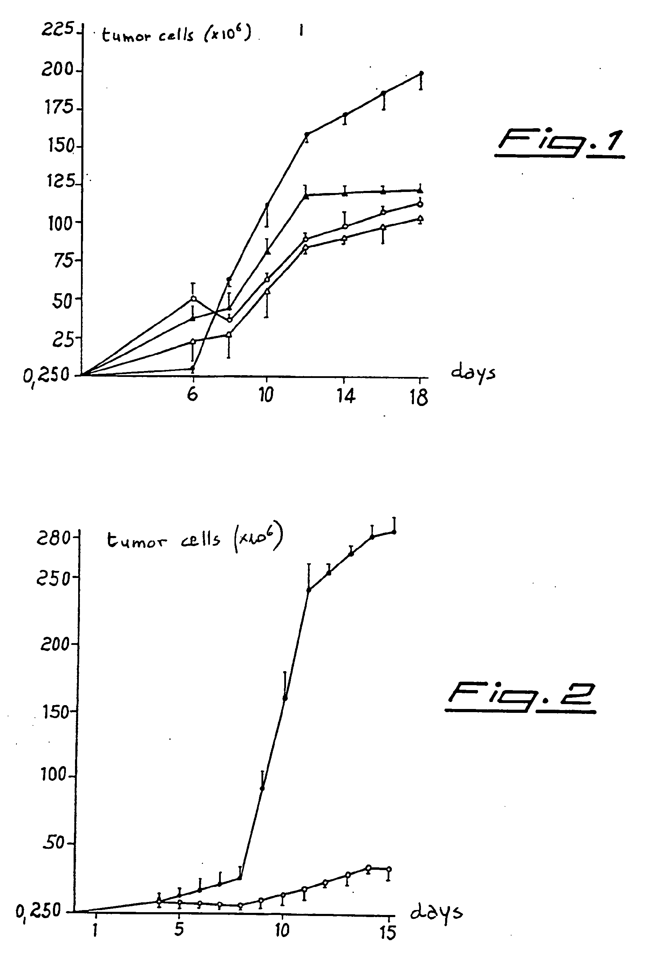

- figures 1 to 7 are graphs showing the different tumor cell proliferation in treated and non-treated rats.

### Description of the preferred embodiments

### Example I - Extract with acids (1 N HClO₄)

Veal liver homogenate (5 g) is dispersed in 10 ml of distilled water, then 10 ml of 2 N HClO₄ are added drop wise in 20 min under stirring at 4°C. stirring is continued for 30 min, and the mixture is centrifuged at 100,000 x g for 20 min at 4°C. The supernatant is dialyzed against running water and then against distilled water all night. It is concentrated by a factor of 5 with Amicon PM 10, and a threefold volume of 4 M KCl in 0.05 M phosphate buffer at pH 7.5 is added. The mixture is stirred at 4°C for 24 h and then centrifuged at 100,000 x g for 1 h at 4°C. The supernatant is dialyzed against phosphate-buffered saline (PBS) and centrifuged again. Finally, the extract is concentrated by ultrafiltration through a membrane with a cutoff of 10,000 daltons up to a protein concentration of 1 mg/ml.

### Example II - Extraction with detergents (PBS + Nonidet P40)

Veal liver (103 g obtained from a freshly killed animal) is cut in small pieces and homogenized in a Waring blender in 260 ml of 0.14 M PBS, pH 7.2, and 0.5% (v/v) Nonidet P40. Homogenization is done at 11,400 rpm for 2 min (alternating 30 s of treatment with 30 s of rest). Protein assays done at various phases of the treatment demonstrated that cell lysis was complete after 2 min. The sample was agitated for 45 min at 4°C and then centrifuged for 90 min at 4°C in a Sorvall SS-34 rotor at 20,500 rpm (50,000 x g). The supernatant (about 300 ml) was then subjected to dialysis at 4°C against 4 L of 0.14 M PBS, pH 7.2, with three changes in the dialysis phase (one change every 8 h). Membranes with a cutoff of 10 kDa were used for the dialysis.

The sample thus obtained, with a volume of 310 ml, was subjected to protein assay according to the method of Lowry, using bovine serum albumin as the calibration protein. A titer of 40.8 ± 2.2 mg protein/ml of solution was obtained. An aliquot (150 ml) of the solution was diluted to 1200 ml by addition of 0.14 M PBS at pH 7.2 and then frozen. The remaining part of the sample was frozen as such.

With the procedures of examples I and II, MHC antigens and their associated molecules with a high molecular weight (from 10,000 to 50,000) were obtained. The liver extract thus obtained, designated AIM-3, was used for in vitro and in vivo tests.

For the in vivo tests, two routes of administration were used: subcutaneous and local. The daily administration to human subjects of 4 ml of the preparation obtained with example II generally gave positive results in about 4 weeks. Neutralization of the MHC proteins by antibodies against the same MHC molecules was avoided by varying every week the origin of the extract, i.e., by using extracts of different batches obtained from tissues of the same species, or of different species.

In any case, different routes of administration can be used, such as parenteral or by aerosol. The pharmaceutical composition will consequently contain vehicles and inert substances that are pharmaceutically acceptable and chosen from those known in the technique, as a function of the chosen administration route.

The invention is now described in greater detail with reference to the following examples and relevant Figs. 1-7. In vitro experiments

### Example A

Molt 4 cells were inoculated with the extract of example I. The inoculated cells produced tissue necrosis factor (TNF) in the supernatant only at 24 h after inoculation, with concentrations clearly superior to those found in untreated cells (1000 pg/ml compared to 300 pg/ml).

### In vivo experimentation

Fischer inbred rats weighing 150-175 g were used. The tumor was induced by inoculation into the pleural cavity of about 250,000 Yoshida AH-130 cells. Such a dose allowed observation of tumor growth for about 18 days before the death of the animal.

Treatment with the composition of the invention, as obtained in example I, was then given in doses of 0.025 mg/kg/day in the pleura, peritoneum or subcutaneously according to the following examples.

### Example B

Some of the inoculated rats were treated with the aforementioned dose of the extract of example I (but obtained from goat liver) in the pleura (A), peritoneum (O) or subcutaneously (Δ) from the 4th to 8th day and then from the 12th to 16th day from cell inoculation (Fig. 1). The data (mean ± SEM of 7 experiments) showed that starting from the 8th day the number of intrapleural tumor cells was significantly less in treated rats than in controls (●) treated with a physiological solution.

### Example C

In this case, the extract of example I was administered, with daily treatment from the 4th to 15th day. Fig. 2 shows that the reduction in growth of tumor cells in treated rats (O) with respect to controls (●).

### Example D

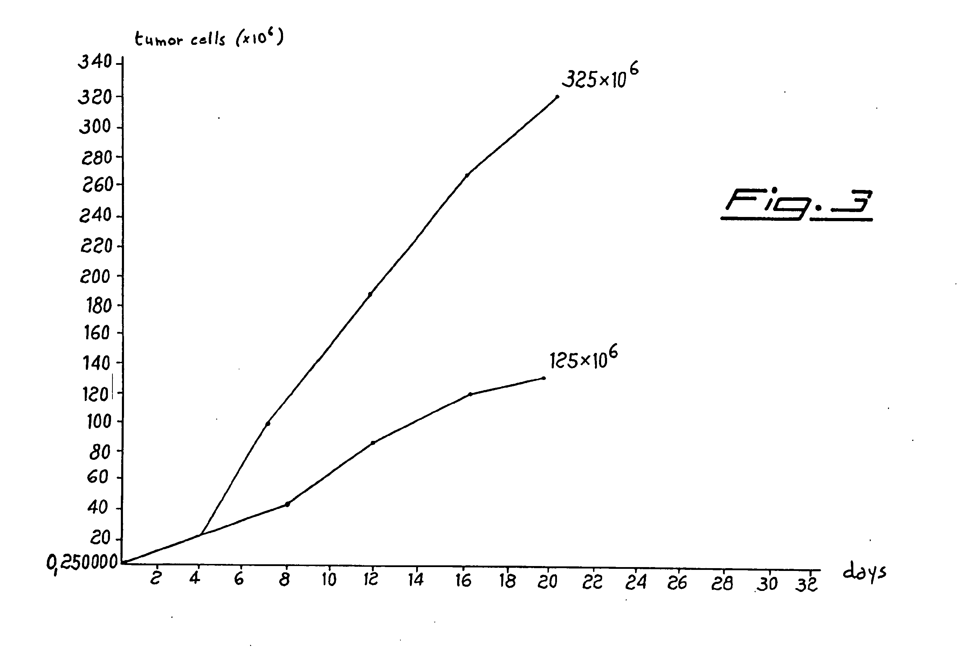

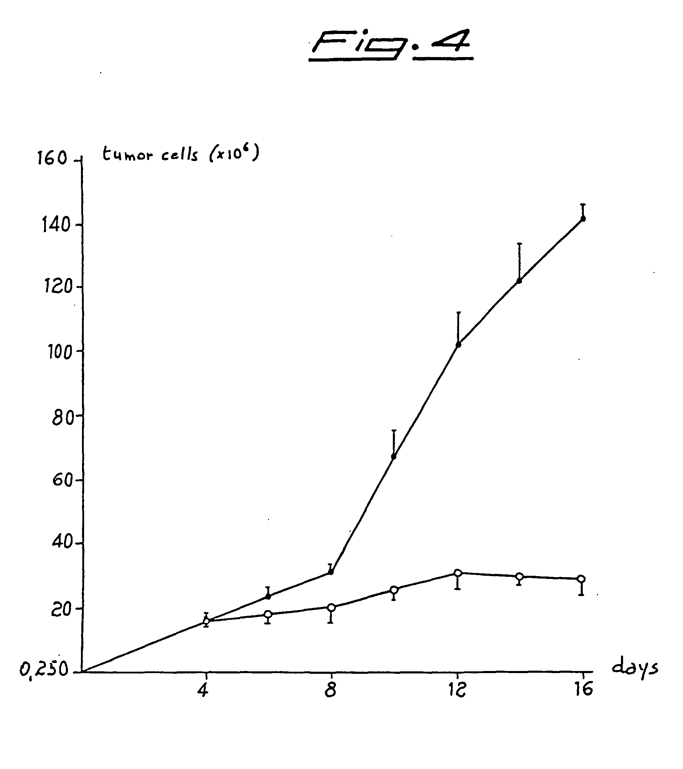

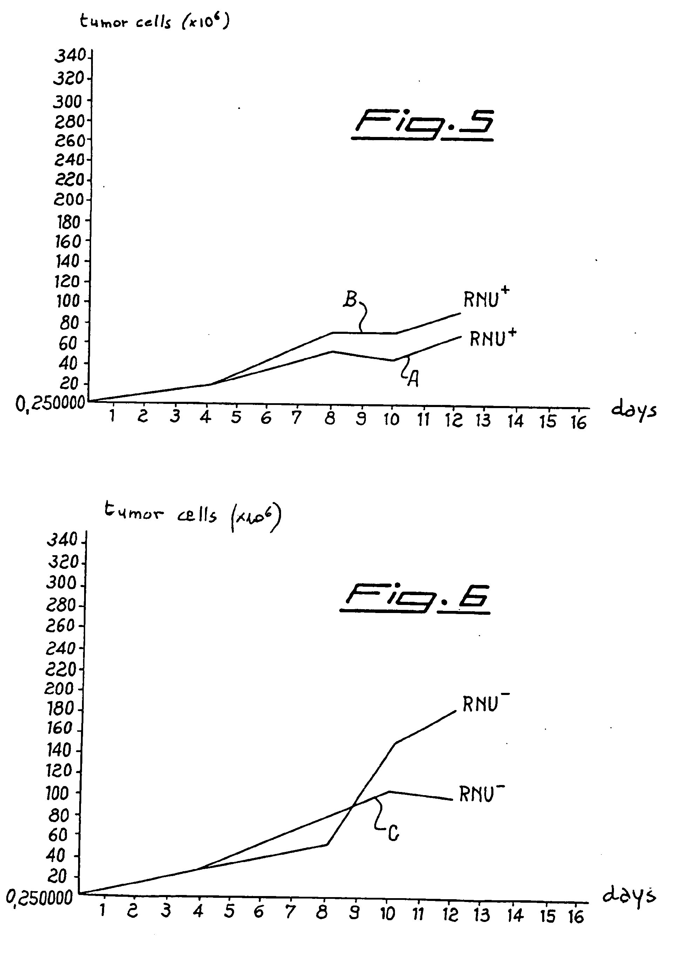

Two extracts were prepared according to example I from two different batches of veal liver homogenate. The first batch was administered to inoculated rats from the 6th to 14th day, and the second batch was administered from day 15 to day 21. The results showed that the tendency of tumor cells to proliferate diminished when the extract was varied (Fig. 3). The controls (upper curve) were treated with a physiological solution as in previous examples.

### Example E

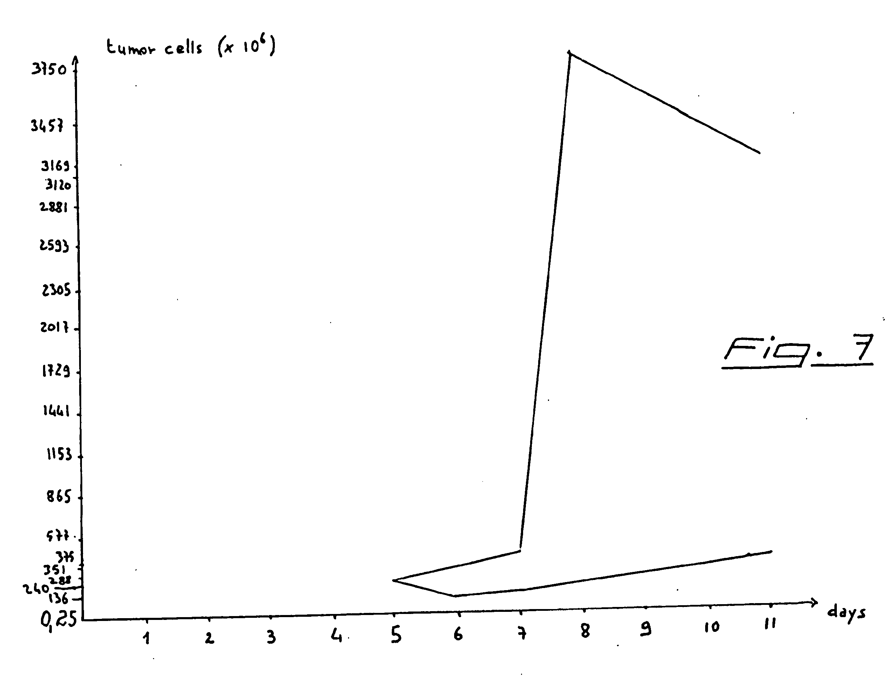

In this experiment, an extract obtained as in example I from goat liver was administered from day 5 to day 10, and the same type of extract but obtained from veal liver was administered from day 11 to day 16. The results showed a marked reduction in cell growth starting from day 8 in treated rats (O) with respect to controls (●) and also with respect to the growth observed in treated rats of the previous examples (Fig. 4).

### Example F

In this case, NIH-RNU⁺ rats (Fig. 5) and NIH-RNU⁻ rats (i.e., without the thymus) (Fig. 6) were used, and the extract of example II was administered. Fig. 5 shows the behavior of treated whole rats (curve A) and corresponding controls (curve B). Fig. 6 shows that treatment with the MHC molecules of the extract obtained according to the invention did not determine a reduction in tumor cell proliferation up to day 8, since the nude rats do not have T lymphocytes. Starting from day 9 in treated rats (curve C), there was a reduction in the growth of neoplastic cells caused by the activation of tumor specific B lymphocytes, and by stimulation of other effector cells.

Although a complete explanation of the action mechanism of the active ingredient of the present invention is not given herein, it is believed to be based on the presence of MHC antigens and molecules, which are highly immunogenic. The efficacy of such molecules is the result of the activation, by MHC proteins, of anergic lymphocytes of the tumor-bearing subject. Tumor cells normally elude the attack of T lymphocytes since the latter, although having recognized the tumor antigens, do not receive a second signal because the tumor cells are devoid of adequate co-stimulating molecules. The molecules codified by a foreign MHC bind to T lymphocyte receptors by a mechanism analogous to that which occurs in transplant rejection and set in motion a series of biochemical events that lead to the destruction of the tumor cells. In rats devoid of T lymphocytes, to which tumor cells were inoculated, the MHC proteins complete the stimulation of B lymphocytes, which - although having recognized the tumor antigen - in the absence of helper T lymphocytes would remain inactive. The results of the following experiments confirm such a hypothesis.

### Example G

Some of the inoculated rats were treated with the aforementioned dose of the extract of example II. Since no appreciable growth of tumor cells was observed, the test was repeated according to the following example.

### Example H

some of the inoculated rats were treated with the previously cited dose starting from the 5th rather than the 4th day, administering only one type of extract. Controls were treated with physiological saline. The results are shown in Fig. 7.

The possible action of the extract of example II against viral agents, particularly HIV, was also investigated. The following in vitro experimentation was carried out.

### Example III

The effect of the substance, added to T-lymphoblastic cell cultures contemporaneously or at 4, 8 and 24 h before HIV infection, on viral replication was evaluated. An aliquot (100 ng) of the substance was added to cell suspensions (about 800,000 cells/ml of viral suspension) of MOLT 4 and CEM cells. The cell suspensions were infected contemporaneously and after 4, 8 and 24 h of preincubation at 37°C in 5% CO₂ of the substance under examination with 100 µl of viral suspension containing 1 x 10⁴ TICD₅₀/ml of HIV.

Evaluation of the inhibitory effect was done by comparing the replicative levels of HIV, measured by determination of the viral P24 antigen or inverse transcriptase activity in the supernatant of pretreated cells with respect to control cells infected at the same times only with the viral suspension. Determinations of viral replication were done at 3, 6, 9, 12 and 15 days from the viral infection. Our results showed that the contemporaneous addition of 100 ng of the substance and viral particles to the cell suspensions did not determine any inhibition of viral replication. A reduction in viral replication was observed only following pretreatment of the cells with the AIM substance for 4 and 8 h before the HIV infection. Such an inhibitory effect was most evident in the first replicative phases of HIV, at 3 and 6 days from the infection, with inhibition values of 60-70%. The inhibitory effect had diminished when viral replication was assayed at 9 days from infection and reached values of 30-20% at 12-15 days from infection. Instead, no inhibitory effect was observed when the cell cultures were pretreated for 24 h before addition of the virus.

## Claims

1. Use of Major Histocompatibility Complex (MHC) xenoantigens in the manufacture of a medicament for the treatment of cancer.

2. The use according to claim 1 wherein said MHC xenoantigens are extracted from animal tissues, serum or cells.

3. The use according to claim 1 or 2 wherein said MHC xenoantigens have a molecular weight between 10,000 and 50,000 daltons.

4. The use according to any one of claims 1 to 3 wherein MHC xenoantigens from different species are to be alternately administered.

5. The use according to any one of claims 1 to 4 wherein said MHC xenoantigens are obtainable as an extract of animal tissues, cells or sera by the use of detergents and have a molecular weight of more than 10,000 daltons.

6. Use according to claim 5 wherein MHC xenoantigens are susceptible to be extracted from goat, veal or pig liver or bovine red blood cells.

## Patentansprüche

1. Verwendung von Haupthistokompatibilitätskomplex (MHC)-Xenoantigenen bei der Herstellung eines Medikamentes für die Behandlung von Krebs.

2. Verwendung gemäß Anspruch 1, wobei die MHC-Xenoantigene aus Tiergeweben, -serum oder -zellen extrahiert werden.

3. Verwendung gemäß Anspruch 1 oder 2, wobei die MHC-Xenoantigene ein Molekulargewicht zwischen 10000 und 50000 Dalton haben.

4. Verwendung gemäß einem der Ansprüche 1 bis 3, wobei die MHC-Xenoantigene aus verschiedenen Spezies abwechselnd zu verabreichen sind.

5. Verwendung gemäß einem der Ansprüche 1 bis 4, wobei die MHC-Xenoantigene als ein Extrakt aus Tiergeweben, -zellen oder -sera durch die Verwendung von Detergenzien erhältlich sind und ein Molekulargewicht von mehr als 10000 Dalton haben.

6. Verwendung gemäß Anspruch 5, wobei die MHC-Xenoantigene einer Extraktion aus Ziegen-, Kalbs- oder Schweineleber oder aus Rindererythrozyten zugänglich sind.

## Revendications

1. Utilisation de xénoantigènes du CMH (complexe majeur d'histocompatibilité) dans la fabrication d'un médicament conçu pour le traitement d'un cancer.

2. Utilisation conforme à la revendication 1, lesdits xénoantigènes de CMH étant tirés de tissus, de sérums ou de cellules d'animaux.

3. Utilisation conforme à la revendication 1 ou 2, lesdits xénoantigènes de CMH présentant un poids moléculaire de 10 000 à 50 000 daltons.

4. Utilisation conforme à l'une des revendications 1 à 3, des xénoantigènes de CMH de différentes espèces devant être administrées en alternance.

5. Utilisation conforme à l'une des revendications 1 à 4, lesdits xénoantigènes de CMH pouvant être obtenus, au moyen de détergents, sous forme d'extraits de tissus, de cellules ou de sérums d'animaux, et présentant un poids moléculaire de plus de 10 000 daltons.

6. Utilisation conforme à la revendication 5, lesdits xénoantigènes de CMH pouvant être tirés de foie de chèvre, de veau ou de porc ou de globules rouges de bovin.
